# EUROPÄISCHE PATENTSCHRIFT

(11) **EP 2 598 182 B1**
(45) Veröffentlichungstag und Bekanntmachung des Hinweises auf die Patenterteilung: **15.03.2017**
(21) Anmeldenummer: 11732346.9
(22) Anmeldetag: 08.07.2011
(51) Int. Cl.: A61M 1/00

(54) **ABSAUGPUMPENSYSTEM**
SUCTION PUMP SYSTEM
SYSTÈME DE POMPE D'ASPIRATION

(30) Priorität: 30.07.2010 CH 12552010
(43) Veröffentlichungstag der Anmeldung: 05.06.2013
(73) Patentinhaber: Medela Holding AG, 6340 Baar (CH)
(72) Erfinder: EHLERT, Hilmar, CH-6052 Hergiswil (CH)
(74) Vertreter: Clerc, Natalia
(86) Internationale Anmeldenummer: PCT/CH2011/000159
(87) Internationale Veröffentlichungsnummer: WO 2012/012908

(56) Entgegenhaltungen:
- EP-A2- 1 166 805
- WO-A2-2007/070570
- WO-A2-2007/128156
- US-A1- 2009 005 747

## Beschreibung

### TECHNISCHES GEBIET

Die vorliegende Erfindung betrifft ein Absaugpumpensystem gemäss Oberbegriff des Patentanspruchs 1.

### STAND DER TECHNIK

Zum Absaugen von Körperfluiden aus Körperkavitäten oder Wunden werden im medizinischen Bereich üblicherweise stationäre Absaugsysteme verwendet. Diese Absaugsysteme bestehen im wesentlichen aus einer Saugquelle, insbesondere einer Vakuumpumpe, einem Fluid- oder Sekretsammelbehälter, einem dazwischen angeordneten Wasserschloss sowie Verbindungsleitungen, namentlich einer vom Patienten zum Fluidsammelbehälter führenden Drainage- oder Sekretleitung, einer vom Sammelbehälter zum Wasserschloss führenden Verbindungsleitung und einer das Wasserschloss mit der Saugquelle verbindenden Vakuumleitung.

Es wurde mittlerweile erkannt, dass es für den Heilprozess wichtig ist, dass sich der Patient so schnell wie möglich selber bewegt und das Bett verlässt. Es sind deshalb mittlerweile portable Absaugsysteme bekannt, welche mehrheitlich in der Wunddrainage eingesetzt werden. WO 2007/128156 offenbart eine derartige tragbare Absaugpumpeneinheit. Sie weist ein Gehäuse mit einem darin angeordneten Pumpaggregat sowie einen Fluidsammelbehälter auf. Dieser Behälter ist lösbar mit dem Gehäuse verbunden, indem er in entsprechenden Führungen von zwei vorstehenden Seitenwänden des Gehäuses einschwenkbar gehalten ist. Ein Drainageschlauch lässt sich mit seinem patientenseitigen, als Adapter ausgebildeten Ende in das Gehäuse einstecken und so mit dem Fluidsammelbehälter verbinden.

Derartige Geräte haben sich in der Praxis bewährt. Die Fachwelt arbeitet gezielt daran, eine möglichst kleine, kompakte und leise Pumpe für eine Vielzahl von Anwendungen zu schaffen, welche zudem einfach bedienbar sein soll.

WO 2007/070570 offenbart ein System zum Sammeln von abgesaugtem Fluid während einer medizinischen Behandlung. Das System weist ein Gehäuse mit einer Pumpe und zwei Fluidsammelbehältern auf. Jeder der Behälter ist über eine eigene Drainageleitung mit dem Patienten verbunden. Die Pumpe ist derart mit Ventilen und Reglern versehen, dass sie unterschiedliche Unterdrücke an die zwei Behälter anlegen kann. Das System ist auf Rädern angeordnet.

US 2009/0005747 beschreibt eine fahrbare Drainagepumpeinheit mit Vakuumpumpe und optionaler externer Vakuumquelle sowie mit zwei oder mehr Behältern. Separate Drainageleitungen können an verschiedenen Stellen Körperflüssigkeiten absaugen.

EP 1 166 805 zeigt ein Mehrfachdrainagesystem mit mehreren Behältern, die in Gruppen in Serie zusammengeschlossen sind Von jeder Gruppe geht eine separate Drainageleitung zum Patienten.

Keine praktikable Lösung wird jedoch für Patienten angeboten, bei welchen an verschiedenen Stellen Körperfluide abgesaugt werden sollen. Wird eine einzige Pumpe mit einem verzweigten Schlauch verwendet, so besteht die erhöhte Gefahr, dass der Schlauch im Bereich des Y-Verbindungsstücks verstopft wird. Werden mehrere Geräte eingesetzt, so erhöht dies die Kosten der Behandlung und das Pflegepersonal muss eine Vielzahl von Geräten bedienen und überwachen.

### DARSTELLUNG DER ERFINDUNG

Es ist deshalb eine Aufgabe der Erfindung, einAbsaugpumpensystem zu schaffen, welches zum gleichzeitigen Absaugen von Körperfluiden eines Patienten an verschiedenen Stellen geeignet ist.

Diese Aufgabe löst ein Absaugpumpensystem mit den Merkmalen des Patentanspruchs 1.

Das erfindungsgemässe Absaugpumpensystem zum Absaugen von Körperfluiden eines Patienten weist ein Gehäuse, mindestens ein Pumpaggregat und mindestens einen Fluidsammelbehälter auf, welcher über eine Vakuumleitung mit dem Pumpaggregat verbunden ist. Das mindestens eine Pumpaggregat ist im Gehäuse angeordnet und das Gehäuse weist mehr als eine Aufnahme zur lösbaren und gleichzeitigen Halterung des Fluidsammelbehälters auf.

Da mehr als ein Fluidsammelbehälter mit dem Gehäuse verbindbar ist, kann mehr als ein Schlauch verwendet werden. Diese Schläuche können somit an verschiedene Stellen des Patienten hin geführt und in unterschiedlichen Körperkavitäten oder an unterschiedlichen Orten derselben Körperkavität Fluide absaugen.

Die Fluidsammelbehälter lassen sich auf bekannte Art und Weise im oder am Gehäuse befestigen. Sie können insbesondere einschwenkbar, einschiebbar oder über mittelbare Verbindungselemente am Gehäuse positionierbar und befestigbar sein.

Unter Fluiden werden hier unter anderem alle Sorten von Körperflüssigkeiten, Sekreten und Fett verstanden, wie sie beispielsweise während Operationen, in der Wunddrainage, der Thoraxdrainage und bei der Fettabsaugung anfallen können.

Das erfindungsgemässe Absaugpumpensystem lässt sich insbesondere für Cardio-Drainagen und für Pleura-Drainagen einsetzen. Dabei lassen sich beide Arten von Drainagen gleichzeitig mit demselben erfindungsgemässen System durchführen.

Vorzugsweise ist das System tragbar ausgebildet.

Vorteilhaft ist, dass durch die Erhöhung der Anzahl der Fluidsammelbehälter die Kapazität für die zu sammelnde Körperfluidmenge erhöht ist. Da für jeden Ort bzw. jede abzusaugende Körperkavität ein eigener Fluidsammelbehälter zur Verfügung gestellt werden kann, lässt sich die Art und die Menge des abgesaugten Fluids auch besser überwachen. Es wird nicht mit Fluiden aus anderen Absaugorten gemischt. Es ist jedoch nach wie vor möglich, mehrere Absaugorte in Gruppen zusammen zu fassen und ihre abgesaugten Fluide in denselben Fluidsammelbehälter zu leiten.

Vorzugweise ist jedoch jeder Fluidsammelbehälter über einen eigenen Drainageschlauch mit dem Patienten verbunden. Jeder Drainageschlauch ist dabei vorzugsweise mit einem eigenen Fluidsammelbehälter verbunden, so dass im mathematischen Sinne eine ein-eindeutige Verbindung zwischen Fluidsammelbehälter und Körperkavität geschaffen ist. Dies erleichtert dem medizinischen Fachpersonal die Analyse und Überwachung, da für jede Fluidsammelstelle anhand des zugehörigen Fluidsammelbehälters festgestellt werden kann, welche Art Flüssigkeit und in welchen Mengen diese Flüssigkeit anfällt.

Dank dem erfindungsgemässen Absaugpumpensystem sind somit keine Verzweigungen der Schläuche und somit keine Y-Verbindungsstücke von Schläuchen mehr notwendig. Das Risiko, dass sich die Schläuche verstopfen, wie dies insbesondere an den Y-Verbindungsstücken gehäuft geschieht, ist dadurch minimiert.

In einer bevorzugten Ausführungsform sind die am Gerät angeordneten Aufnahmen zur Aufnahme der Fluidsammelbehälter identisch ausgebildet. Das System kann identisch und gleich gross ausgebildete Fluidsammelbehälter verwenden. Es kann aber auch unterschiedliche Fluidsammelbehälter aufnehmen. Die Unterschiede können beispielsweise in der Form, in der Grösse, in der Art der Filter, in der Art des Fluidklumpmittels oder in der Art der im oder am Behälter angeordneten Sensoren bestehen.

Das System kann jedoch auch mit unterschiedlich ausgebildeten Aufnahmen versehen sein, in welche jeweils nur entsprechend geformte Fluidsammelbehälter aufgenommen werden können.

Vorzugsweise sind die Aufnahmen nebeneinander angeordnet.

In einer einfachen Ausführungsform ist nur ein einziges Pumpaggregat mit einer einzigen Vakuumpumpe im Gehäuse angeordnet. Diese Pumpe ist über im Gehäuse verlaufende interne oder ausserhalb des Gehäuses verlaufende externe Vakuumleitungen mit den Fluidsammelbehältern verbunden. Diese einfache Ausführungsform weist den Vorteil auf, dass sie kostengünstig, relativ leicht und relativ klein ist.

In anderen Ausführungsformen sind mindestens zwei Pumpaggregate bzw. Vakuumpumpen im Gehäuse angeordnet. Jede dieser Vakuumpumpen ist über interne oder externe Vakuumleitungen mit mindestens einem Fluidsammelbehälter verbunden. Auch hier ist eine ein-eindeutige Verbindung bevorzugt, so dass jedem Fluidsammelbehälter eine eigene Pumpe zugeordnet ist. Dieses System weist den Vorteil auf, dass bei Ausfall einer Pumpe auf eine andere in demselben Gehäuse gewechselt werden kann.

Es ist auch möglich, dass eine Pumpe mehrere Fluidsammelbehälter mit Unterdruck beaufschlägt.

Im Gehäuse ist mindestens eine Steuerelektronik, auch PCB (Printed Circuit Board) genannt, angeordnet. Diese Steuerelektronik dient zur Betreibung des Pumpaggregats, wobei sie von allfälligen Sensoren erhaltene Signale verarbeitet und verwendet. In einer einfachen Ausführungsform ist diese Steuerelektronik mehreren bzw. allen Pumpaggregaten zugeordnet und betreibt somit mehrere bzw. alle Vakuumpumpen. Dies reduziert die Kosten. In anderen Ausführungsformen ist jedes Pumpaggregat, d.h. jede Vakuumpumpe, einer eigenen Steuerelektronik zugeordnet. Es liegt somit wiederum eine ein-eindeutige Zuordnung vor. Es können auch mehrere Gruppen von Pumpaggregaten gebildet sein, welche einer eigenen Steuerelektronik zugeordnet sind. Die verschiedenen Steuerungen können auf derselben Leiterplatte aufgebracht sein. Vorzugsweise weist jedoch jede Steuerung ihre eigene Leiterplatte auf. Werden mehrere Steuerungen verwendet, so können diese auf die entsprechende Applikation, wie z.B. Cardio-Drainage oder Pleura-Drainage, optimiert sein. Des Weiteren ist bei einem Ausfall einer Pumpe bzw. Steuerelektronik die andere Pumpe nach wie vor funktionsfähig.

Das System weist mindestens eine Eingabe- und Anzeigeeinheit auf. Sind mehrere Einheiten vorhanden, so sind sie vorzugsweise jeweils einem einzigen Fluidsammelbehälter und/oder jeweils einem einzigen Pumpaggregat zugeordnet. Sie können jedoch auch Gruppen davon zugeordnet sein.

Vorzugweise ist im Gehäuse mindestens eine Batterie zur Stromversorgung der Elektronik und des Pumpenmotors vorhanden. Es kann eine einzige Batterie für alle Pumpen vorhanden sein oder jeder Pumpe kann eine eigene Batterie zugeordnet sein. Des Weiteren kann auch eine Gruppe von Pumpen mit einer Batterie verbunden sein und eine andere Gruppe einer anderen Batterie. Die Verbindung mit dem öffentlichen Stromversorgungsnetz ist vorzugsweise ebenfalls möglich.

Vorzugsweise sind Module der oben genannten einzelnen Elemente vorhanden, welche zu einem gemeinsamen System in einem gemeinsamen Gehäuse zusammengefasst sind. Diese Module umfassen beispielsweise je ein Pumpaggregat, je einen Fluidsammelbehälter, je eine Steuerelektronik und je eine Eingabe- und Anzeigeeinheit und optional noch je eine Batterie. Vorzugsweise sind zumindest die Eingabe- und Anzeigeeinheit sowie der zugehörige Fluidsammelbehälter so angeordnet, dass ein Benützer eine eindeutige Zuordnung der Eingabe- und Anzeigeeinheiten zum entsprechenden Fluidsammelbehälter bilden kann.

Vorzugsweise weisen alle Module dieselbe Breite auf, so dass eine gleiche Teilung des Gehäuses vorhanden ist. Dadurch lassen sich die einzelnen Module einfach austauschen. Es lässt sich beispielsweise mit denselben Bauteilen ein System zusammenstellen, welches zwei Cardio-Applikationen und eine Pleura-Applikation aufweist, und ein anderes System, welches eine Cardio-Applikation und zwei Pleura-Applikationen aufweist.

Ein grosser Vorteil des erfindungsgemässen Systems ist, dass ein einziges Gerät dem Benützer eine Vielzahl von Möglichkeiten gibt und trotzdem eine einfache Bedienung erlaubt. Der Arzt bzw. das Pflegepersonal kann entscheiden, welche und wie viele der zur Verfügung gestellten Drainagebehälter verwendet werden. Er kann auch die Grösse der Drainagebehälter wählen. Der Arzt bzw. das Pflegepersonal kann entscheiden, wie viele Pumpen und welche Art Pumpen bzw. Steuerung verwendet werden soll. Er kann mit demselben Gerät gleichzeitig Pleura- und Cardio-Drainage und weitere Arten von Drainage durchführen.

Weitere Ausführungsformen sind in den abhängigen Ansprüchen angegeben.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Figur 1: eine schematische Darstellung eines erfindungsgemässen Absaugpumpensystems im Einsatz bei einem Patienten in einer ersten Ausführungsform;
- Figur 2: eine schematische perspektivische Darstellung eines erfindungsgemässen Absaugpumpensystems in einer zweiten Ausführungsform von einer ersten Seite;
- Figur 3: das Absaugpumpensystem gemäss Figur 2 von einer zweiten Seite;
- Figur 4: eine schematische Darstellung des Absaugpumpensystems gemäss Figur 2;
- Figur 5: eine schematische Darstellung des erfindungsgemässen Absaugpumpensystems in einer dritten Ausführungsform und
- Figur 6: eine schematische Darstellung des erfindungsgemässen Absaugpumpensystems in einer vierten Ausführungsform.

### BESCHREIBUNG BEVORZUGTER AUSFÜHRUNGSFORMEN

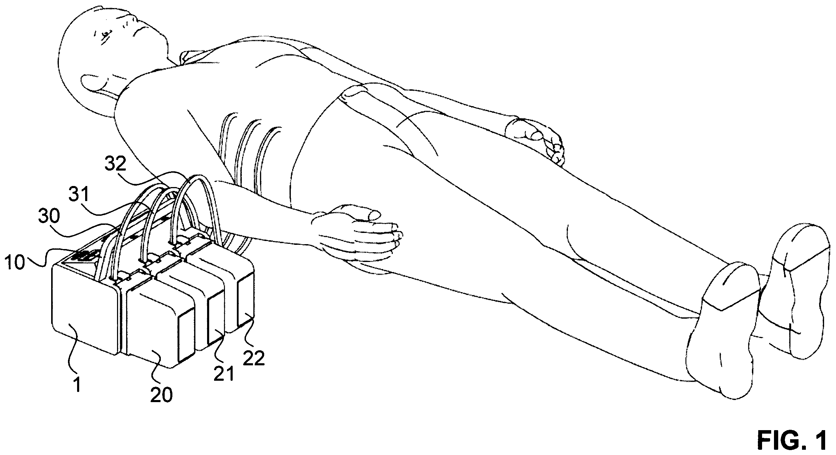

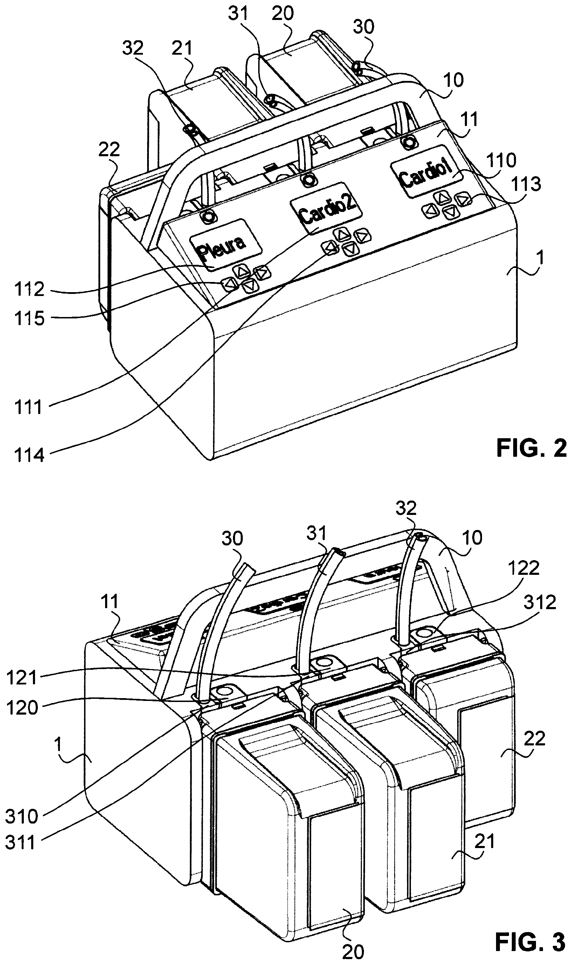

In Figur 1 ist ein erstes Beispiel eines erfindungsgemässen Absaugpumpensystems dargestellt. Es ist als portables Gerät ausgebildet. Es weist ein Gehäuse 1 mit einem zum Tragen des Geräts ausgebildeten Traggriff 10 auf. Im Gehäuse 1 ist mindestens ein Pumpenaggregat, d.h. eine Vakuumpumpe, angeordnet. Am Gehäuse sind Fluidsammelbehälter 20, 21, 22 befestigbar. Hier sind sie mittels in Figur 3 sichtbaren Einrastklinken 120, 121, 122 in ihrer Position gehalten. Vorzugsweise weist das Gehäuse 1 eine Aufnahme auf, in welche die Sammelbehälter 20, 21, 22 in ihrer gewünschten Gebrauchsposition einschiebbar oder einschwenkbar sind. Die Fluidsammelbehälter 20, 21, 22 sind vorzugsweise nebeneinander angeordnet, wobei alle Aufnahmen gleich ausgebildet sind.

Ferner sind Sekretschläuche, hier Drainageschläuche 30, 31, 32 genannt, vorhanden. Diese führten zu einem Patienten. Es können ein- oder mehrlumige Schläuche sein. Dabei weist jeder Schlauch eine Drainageleitung auf. Vorzugsweise ist ferner eine Serviceleitung mit einem kleineren Durchmesser vorhanden, welche beispielsweise zur Spülung der Leitung dient.

Wie in Figur 3 erkennbar ist, sind die pumpenseitigen Enden der Drainageschläuche 30, 31, 32 mit Kopplungsteilen 310, 311, 312 verbunden, welche in das Gehäuse einsteckbar sind und welche dadurch je eine Drainageverbindung mit je einem benachbarten Fluidsammelbehälter 20, 21, 22 erstellt.

Im Gebrauch wird mittels der Vakuumpumpe und einer im Gehäuse 1 verlaufenden Vakuumleitung im jeweiligen Fluidsammelbehälter 20, 21, 22 ein Vakuum bzw. ein Unterdruck erzeugt. Durch die Verbindung des Fluidsammelbehälters 20, 21, 22 mit dem jeweiligen Drainageschlauch 30, 31, 32 wird ein Unterdruck in der Körperkavität des Patienten angelegt. Körperfluid wird durch den Drainageschlauch 30, 31, 32 in den Fluidsammelbehälter 20, 21, 22 gesaugt und dort gesammelt.

Es ist auch möglich, die Drainageschläuche 30, 31, 32 anstatt im Gehäuse 1 direkt in die Fluidsammelbehälter 20, 21, 22 einzustecken. Zudem ist es möglich, dass Drainageschläuche 30, 31, 32 mit dem jeweiligen Fluidsammelbehälter 20, 21, 22 in einer nicht zerstörungsfrei lösbaren Verbindung stehen. Dies ist vorteilhaft, da sowohl Fluidsammelbehälter 20, 21, 22 wie auch Drainageschläuche 30, 31, 32 ein Einwegprodukt sind, welche nach Gebrauch fachgerecht entsorgt werden müssen. Das Gehäuse 1 mit der mindestens einen Vakuumpumpe hingegen ist mehrfach einsetzbar.

Vorzugsweise sind die Drainageschläuche 30, 31, 32, insbesondere die Drainageleitungen unverzweigt, so dass eine 1:1 Verbindung zwischen Fluidsammelbehälter 20, 21, 22 und dem Patienten geschaffen werden kann. Die Schläuche können an verschiedene Stellen des Patientenkörpers zugeführt werden.

Im Beispiel gemäss Figur 1 sind alle Fluidsammelbehälter 20, 21, 22 gleich ausgebildet und weisen insbesondere dieselbe Grösse und dasselbe Fassungsvermögen auf. Im Beispiel gemäss den Figuren 2 und 3 ist einer der Fluidsammelbehälter 22 kleiner und mit kleinerem Fassungsvermögen ausgebildet als die zwei anderen Fluidsammelbehälter 20, 21. Es lassen sich auch andere Grössenverhältnisse wählen und/oder jeder Fluidsammelbehälter lässt sich mit einem anderen Fassungsvermögen bzw. Form ausbilden. Vorzugsweise sind die Aufnahmen des Gehäuses 1 so ausgebildet, dass dieselbe Aufnahme verschieden grosse Fluidsammelbehälter aufnehmen kann.

In den Figuren 2 und 3 ist am Gehäuse 1 eine Konsole 11 erkennbar, auf welcher mindestens eine Eingabe- und Anzeigeeinheit vorhanden ist. Die Konsole 11 ist in diesem Beispiel geneigt ausgebildet. Die Anzeige weist vorzugsweise eine Anzeigefläche (ein Display) 110, 111, 112 auf. Als Eingabefelder 113, 114, 115 lassen sich wie hier dargestellt Eingabetasten, Drehknöpfe oder andere geeignete Mittel verwenden.

Die in den Figuren 1 bis 3 dargestellten Ausführungsbeispiele sind als Module ausgebildet. Die Anzeigefläche 112, das Eingabefeld 115 sowie der Fluidsammelbehälter 22 lassen sich einander eindeutig zuordnen. Dies gilt auch für die übrigen Anzeigeflächen, Eingabefelder und Fluidsammelbehälter. Mindestens die einzelnen äusseren Elemente der Module, d.h. die oben genannten Elemente inkl. die zugehörigen Aufnahmen des Gehäuses 1, sind vorzugsweise nebeneinander angeordnet. Dies erleichtert die Übersicht und die Zuordnung.

Es lassen sich somit, wie dies in den Figuren 2 und 3 erkennbar ist, mit demselben Gerät gleichzeitig verschiedene Drainageapplikationen ausführen. Display 112 zeigt eine Pleura-Drainage, die Displays 110, 111 zeigen zwei verschiedene Cardio-Drainagen.

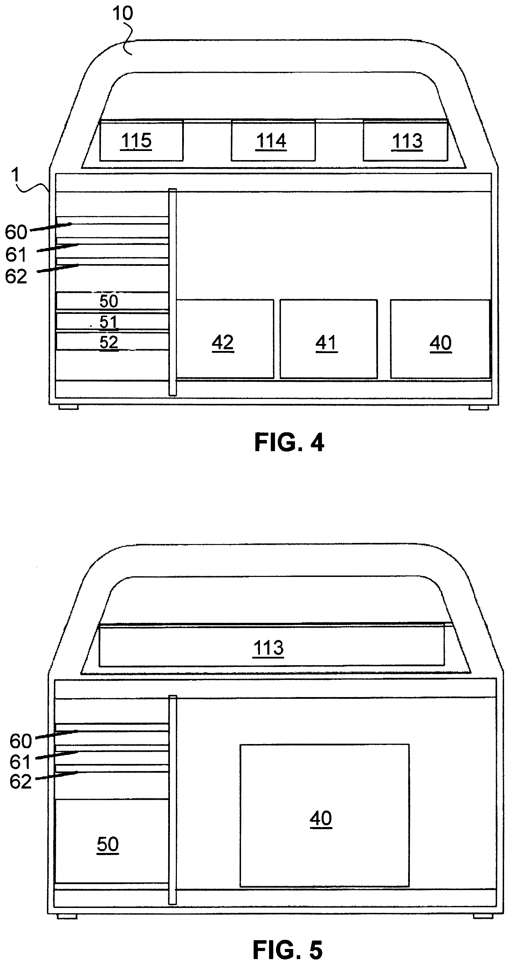

In Figur 4 ist das Innere des Gehäuses 1 schematisch dargestellt. In diesem Ausführungsbeispiel sind drei Pumpaggregate oder Vakuumpumpen 40, 41, 42 vorhanden, welche jeweils mit einer nur dieser Pumpe zugeordneten Steuerelektronik 60, 61, 62 betrieben werden. Auch die Zuordnung der Eingabe- und Anzeigeeinheiten 113, 114, 115 und der hier nicht sichtbaren Fluidsammelbehälter 20, 21, 22 sind ein-eindeutig.

Ferner sind Batterien 50, 51, 52 und/oder Netzteil zum Anschluss an ein externes Stromversorgungsnetz vorhanden, welche in diesem Beispiel ebenfalls ein-eindeutig einem jeweiligen Modul zugeordnet sind.

In Figur 5 ist nur eine Vakuumpumpe 40, nur eine Batterie 50 bzw. nur ein Netzteil und nur eine Anzeige- und Eingabeeinheit 113 für alle Fluidsammelbehälter vorhanden. Es sind jedoch nach wie vor drei Steuerelektronikeinheiten 60, 61, 62 vorhanden. Dadurch lassen sich, wie in den Figuren 2 und 3 dargestellt, nach wie vor gleichzeitig unterschiedliche Drainage-Applikationen durchführen, wobei diese auf demselben Display angezeigt werden und durch dieselbe Vakuumpumpe erzeugt werden.

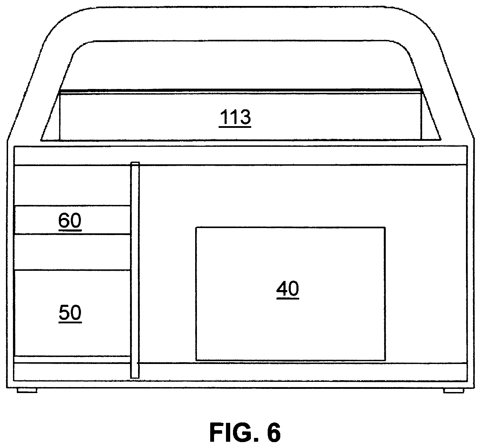

Im Ausführungsbeispiel gemäss Figur 6 ist auch nur eine einzige Steuerelektronik, vorzugsweise nur ein einziges PCB vorhanden.

Weitere Kombinationsarten der einzelnen Elemente des Systems sind möglich. In den beschriebenen Ausführungsformen wurden genau drei Fluidsammelbehälter im gleichen Gehäuse gehalten. Andere Zahlen sind möglich, es können insbesondere zwei, vier oder mehr Behälter sein. Die Anzahl der einzelnen Elemente können beliebig gewählt werden und müssen nicht mit der Anzahl der Elemente eines anderen Typs übereinstimmen. So können beispielsweise zwei Pumpen in Kombination mit drei Behältern verwendet werden.

Das erfindungsgemässe Absaugpumpensystem ermöglicht ein gleichzeitiges Absaugen von Körperfluiden eines Patienten an verschiedenen Stellen. Dabei ist es einfach bedienbar, bietet dem Benutzer eine grosse Flexibilität in der Wahl der Drainage und ermöglicht einen guten Überblick über die gewählten Applikationen.

**Bezugszeichenliste**

| | | | |
|---|---|---|---|
| 1 | Gehäuse | | |
| 10 | Traggriff | 20,21,22 | Fluidsammelbehälter |
| 11 | Konsole | | |
| 110 | erste Anzeigefläche | 30,31,32 | Drainageschlauch |
| 111 | zweite Anzeigefläche | 310 | erstes Kopplungsteil |
| 112 | dritte Anzeigefläche | 311 | zweites Kopplungsteil |
| 113 | erstes Eingabefeld | 312 | drittes Kopplungsteil |
| 114 | zweites Eingabefeld | | |
| 115 | drittes Eingabefeld | 40,41,42 | Pumpaggregat |
| 120 | erste Einrastklinke | 50,51,52 | Batterie |
| 121 | zweite Einrastklinke | 60,61,62 | Steuerelektronik |
| 122 | dritte Einrastklinke | | |

## Patentansprüche

1. Absaugpumpensystem zum Absaugen von Körperfluiden eines Patienten, wobei das Absaugpumpensystem ein Gehäuse (1), mindestens ein Pumpaggregat (40, 41, 42) und mindestens einen Fluidsammelbehälter (20, 21, 22) aufweist, wobei das mindestens eine Pumpaggregat (40, 41, 42) im Gehäuse (1) angeordnet ist und wobei das Gehäuse (1) eine Aufnahme zur lösbaren Halterung des Fluidsammelbehälters (20, 21, 22) aufweist, wobei das Gehäuse (1) mindestens zwei derartige Aufnahmen zur gleichzeitigen Halterung von je einem Fluidsammelbehälter (20, 21, 22) aufweist, **dadurch gekennzeichnet, dass** Pumpaggregat (40, 41, 42), Fluidsammelbehälter (20, 21, 22), Steuerelektronik (60, 61, 62) und Eingabe- und Anzeigeeinheit (110-115) ein Modul bilden, wobei mindestens zwei derartige Module vorhanden sind, und dass die Eingabe- und Anzeigeeinheit (110-115) und der Fluidsammelbehälter (20, 21, 22) eines Moduls einander eindeutig zuordnungsbar sind.

2. Absaugpumpensystem nach Anspruch 1, wobei ferner Drainageleitungen (30, 31, 32) zur Verbindung der Fluidsammelbehälter (20, 21, 22) mit dem Patienten vorhanden sind, wobei jede Drainageleitung (30, 31, 32) mit einem eigenen Fluidsammelbehälter (20, 21, 22) verbunden ist.

3. Absaugpumpensystem nach einem der Ansprüche 1 oder 2, wobei die Aufnahmen identisch ausgebildet sind.

4. Absaugpumpensystem nach einem der Ansprüche 1 bis 3, wobei die Aufnahmen nebeneinander angeordnet sind.

5. Absaugpumpensystem nach einem der Ansprüche 1 bis 4, wobei mindestens zwei Pumpaggregate (40, 41, 42) im Gehäuse (1) angeordnet sind.

6. Absaugpumpensystem nach Anspruch 5, wobei jedem Fluidsammelbehälter (20, 21, 22) ein eigenes Pumpaggregat (40, 41, 42) zugeordnet ist.

7. Absaugpumpensystem nach einem der Ansprüche 1 bis 4, wobei mehrere Fluidsammelbehälter (20, 21, 22) demselben Pumpaggregat (40, 41, 42) zugeordnet sind.

8. Absaugpumpensystem nach Anspruch 7, wobei alle Fluidsammelbehälter (20, 21, 22) demselben Pumpaggregat (40) zugeordnet sind.

9. Absaugpumpensystem nach einem der Ansprüche 1 bis 8, wobei im Gehäuse (1) mindestens eine Steuerelektronik (60, 61, 62) angeordnet ist.

10. Absaugpumpensystem nach Anspruch 9, wobei mehrere Pumpaggregate (40, 41, 42) vorhanden sind und diese derselben Steuerelektronik (60, 61, 62) zugeordnet sind.

11. Absaugpumpensystem nach Anspruch 10, wobei alle Pumpaggregate (40, 41, 42) derselben Steuerelektronik (60, 61, 62) zugeordnet sind.

12. Absaugpumpensystem nach Anspruch 9, wobei jedes Pumpaggregat (40, 41, 42) einer eigenen Steuerelektronik (60, 61, 62) zugeordnet ist.

13. Absaugpumpensystem nach einem der Ansprüche 1 bis 12, wobei mehrere Eingabe- und Anzeigeeinheiten (110- 115) vorhanden sind und diese jeweils einem Fluidsammelbehälter (20, 21, 22) und/oder jeweils einem Pumpaggregat (40, 41, 42) zugeordnet ist.

14. Absaugpumpensystem nach einem der Ansprüche 1 bis 13, Absaugpumpensystem nach einem der Ansprüche 1 bis 13, wobei das System tragbar ausgebildet ist.

## Claims

1. A suction pump system for aspirating body fluids from a patient, which suction pump system has a housing (1), at least one pump unit (40, 41, 42), and at least one fluid collection container (20, 21, 22), the at least one pump unit (40, 41, 42) being arranged in the housing (1), and the housing (1) having a fixture for releasably holding the fluid collection container (20, 21, 22), wherein the housing (1) has at least two such fixtures for simultaneously holding in each case one fluid collection container (20, 21, 22), **characterized in that** pump unit (40, 41, 42), fluid collection container (20, 21, 22), control electronics unit (60, 61, 62) and input and display unit (110-115) form a module, and at least two such modules are present, and **in that** the input and display unit (110-115) and the fluid collection container (20, 21, 22) of a module can be unambiguously assigned to one another.

2. The suction pump system as claimed in claim 1, in which drainage lines (30, 31, 32) for connecting the fluid collection containers (20, 21, 22) to the patient are also present, and each drainage line (30, 31, 32) is connected to its own fluid collection container (20, 21, 22).

3. The suction pump system as claimed in either of claims 1 and 2, in which the fixtures are designed identically.

4. The suction pump system as claimed in one of claims 1 through 3, in which the fixtures are arranged next to one another.

5. The suction pump system as claimed in one of claims 1 through 4, in which at least two pump units (40, 41, 42) are arranged in the housing (1).

6. The suction pump system as claimed in claim 5, in which each fluid collection container (20, 21, 22) is assigned its own pump unit (40, 41, 42).

7. The suction pump system as claimed in one of claims 1 through 4, in which several fluid collection containers (20, 21, 22) are assigned to the same pump unit (40, 41, 42).

8. The suction pump system as claimed in claim 7, in which all the fluid collection containers (20, 21, 22) are assigned to the same pump unit (40).

9. The suction pump system as claimed in one of claims 1 through 8, in which at least one control electronics unit (60, 61, 62) is arranged in the housing (1).

10. The suction pump system as claimed in claim 9, in which several pump units (40, 41, 42) are present, and these are assigned to the same control electronics unit (60, 61, 62).

11. The suction pump system as claimed in claim 10, in which all the pump units (40, 41, 42) are assigned to the same control electronics unit (60, 61, 62).

12. The suction pump system as claimed in claim 9, in which each pump unit (40, 41, 42) is assigned to its own control electronics unit (60, 61, 62).

13. The suction pump system as claimed in one of claims 1 through 12, in which several input and display units (110-115) are present, and these are each assigned to a fluid collection container (20, 21, 22) and/or to a pump unit (40, 41, 42).

14. The suction pump system as claimed in one of claims 1 through 13, in which the system is designed to be portable.

## Revendications

1. Un système de pompe d'aspiration pour l'aspiration de fluides corporels d'un patient, où le système de pompe d'aspiration présente un boîtier (1), au moins un ensemble de pompe (40, 41 42) et au moins un réservoir collecteur de fluide (20, 21, 22), où l'au moins un ensemble de pompe (40, 41 42) est disposé dans le boîtier (1), et où le boîtier (1) présente un logement pour le maintien amovible du réservoir collecteur de fluide (20, 21, 22), où le boîtier (1) présente au moins deux logements de ce type pour le maintien simultané de respectivement un réservoir collecteur de fluide (20, 21, 22), **caractérisé en ce que** l'ensemble de pompe (40, 41, 42), le réservoir collecteur de fluide (20, 21, 22), l'électronique de commande (60, 61, 62) et l'unité d'entrée et d'affichage (110-115) forment un module, où au moins deux modules de ce type sont présents et que l'unité d'entrée et d'affichage (110 à 115) et le réservoir collecteur de fluide (20, 21, 22) d'un module peuvent être attribués l'un à l'autre de manière non ambiguë.

2. Le système de pompe d'aspiration selon la revendication 1, où d'avantage des conduites de drainage (30, 31, 32) existent pour une connexion des réservoirs collecteurs de fluide (20, 21, 22) au patient, où chaque conduite de drainages (30, 31, 32) est connectée à son propre réservoir collecteur de fluide (20, 21, 22).

3. Le système de pompe d'aspiration selon une de revendications 1 ou 2, où les logements sont formés de manière identique.

4. Le système de pompe d'aspiration selon une des revendications 1 à 3, où les logements sont disposés l'un à côté de l'autre.

5. Le système de pompe d'aspiration selon une des revendications 1 à 4, où au moins deux ensembles de pompe (40, 41, 42) sont disposés dans le boîtier (1).

6. Le système de pompe d'aspiration selon la revendication 5, où chaque réservoir collecteur de fluide (20, 21, 22) est attribué à son propre ensemble de pompe (40, 41, 42).

7. Le système des pompes d'aspiration selon une des revendications 1 à 4, où plusieurs réservoirs collecteurs de fluide (20, 21, 22) sont attribués au même ensemble de pompe (40, 41, 42).

8. Le système de pompe d'aspiration selon la revendication 7, où tous les réservoirs collecteurs de fluide (20, 21, 22) sont attribués au même ensemble de pompe (40).

9. Le système de pompe d'aspiration pour selon une des revendications 1 à 8, où au moins une électronique de commande (60, 61, 62) est disposée dans le boîtier (1).

10. Le système des pompe d'aspiration selon la revendication 9, où plusieurs ensembles de pompe (40, 41, 42) existent et ceux-ci sont attribués à la même électronique de commande (60, 61, 62).

11. Le système des pompe d'aspiration selon la revendication 10, où tous les ensembles de pompe (40, 41, 42) sont attribués à la même électronique de commande (60, 61, 62).

12. Le système des pompes d'aspiration selon la revendication 9, où chaque ensemble de pompe (40, 41, 42) est attribué à sa propre électronique de commande (60, 61, 62).

13. Le système de pompe d'aspiration selon une des revendications 1 à 12, où plusieurs unités d'entrée et d'affichage (110 à 115) existent et celles-ci sont attribuées à respectivement un réservoir collecteur de fluide (20, 21, 22) et/ou à respectivement un ensemble de pompe (40, 41, 42).

14. Le système de pompe d'aspiration pour selon une des revendications 1 à 13, où le système est formé de manière portable.
